Europäisches Patentamt

(19) European Patent Office

Office européen des brevets

(11) Veröffentlichungsnummer: **0 133 444**
**B1**

(12) **EUROPÄISCHE PATENTSCHRIFT**

(45) Veröffentlichungstag der Patentschrift :
07.09.88

(21) Anmeldenummer : 84102362.5

(22) Anmeldetag : 05.03.84

(51) Int. Cl.⁴ : **A 61 K 49/04, C 01 F 11/46**

(54) **Verfahren zur Herstellung von als schattengebende Komponente in Röntgenkontrastmitteln geeignetes Bariumsulfat mit erhöhter Fliessfähigkeit und Dichte, nach diesem Verfahren erhaltenes Produkt und daraus hergestellte Röntgenkontrastmittel.**

(30) Priorität : 04.08.83 IT 2242583

(43) Veröffentlichungstag der Anmeldung :
27.02.85 Patentblatt 85/09

(45) Bekanntmachung des Hinweises auf die Patenterteilung : 07.09.88 Patentblatt 88/36

(84) Benannte Vertragsstaaten :
AT BE CH DE FR GB IT LI NL SE

(56) Entgegenhaltungen :
DE-A- 2 703 600
DE-C- 700 065
CHEMICAL ABSTRACTS, Band 84, Nr. 14, 5. April 1976, Seite 139, Zusammenfassung Nr. 92337a, Columbus, Ohio, US; & SU-A- 481 544 (T.G. AKHMETOV) 25-08-1975
Winnacker-Küchler, Chem. Technologie, Bd. 2, pp. 148-155

(73) Patentinhaber : BRACCO INDUSTRIA CHIMICA Società per Azioni
Via E. Folli, 50
I-20134 Milano (IT)

(72) Erfinder : Felder, Ernst, Prof. Dr.
Via Ceresio 49
CH-6826 Riva S. Vitale (CH)
Erfinder : Zingales, Maria, Dr.

verstorben (IT)

(74) Vertreter : Zutter, Hans Johann Niklaus
EPROVA AG Forschungsinstitut Im Laternenacker 5
CH-8200 Schaffhausen (CH)

**Beschreibung**

Die vorliegende Erfindung betrifft gesintertes Bariumsulfat, geeignet zur Verwendung von Röntgenkontrastmitteln.

Die Erfindung umfasst auch Röntgenkontrastmittel, insbesondere geeignet zur Doppelkontrastdarstellung des Magens bzw. der Magenschleimhaut, die durch Hochtemperatur gesintertes Bariumsulfat mit erhöhter Schüttdichte enthalten, deren wässerige Suspensionen eine stark erhöhte Fliessfähigkeit aufweisen.

Bariumsulfat ist nach wie vor die bedeutendste schattengebende Komponente in Röntgenkontrastmitteln, die zur Darstellung des Magen-Darm-Traktes verwendet werden.

Zahlreiche neue Bariumsulfat-Präparate und solche betreffende Patente belegen die unübertroffene prinzipielle Eignung dieser schattengebenden Verbindung.

Für die praktische Anwendung werden Bariumsulfat-Präparate angestrebt, die höchste Strahlenabsorption mit guter Fliessfähigkeit verbinden, damit sie in alle Körperteile und Falten eindringen und eine ausreichende Schattendichte selbst bei geringer Schichtdicke erreicht werden kann.

Als Stand der Technik für allgemein anwendbare Präparate kann etwa die deutsche Offenlegungsschrift (DE-OS) 2 703 600 (vom 03.08.1978) (Fredi Fischer) herangezogen werden. Demnach soll ein als Röntgenkontrastmittel geeignetes Bariumsulfat eine Schüttdichte von mindestens 1.2 g/ml, vorzugsweise von 1.6 bis 1.7 g/ml aufweisen.

Noch höhere Anforderungen hinsichtlich Dichte und Fliessfähigkeit werden an Röntgenkontrastmittel gestellt, welche für die Doppelkontrastdarstellung des Magens bzw. der Magenschleimhaut geeignet sind. Die Doppelkontrastdarstellung ist eine in den letzten Jahren entwickelte Methode zur Auffindung von Magenschleimhautläsionen, welche gewöhnlich die erste Stufe einer Krebserkrankung der Magenwand darstellen. R.E. Miller and J. Skucas, Radiographic Contrast Agents, University Park Press 1977, Seiten 144-146.

Durch die Früherkennung solcher Läsionen lassen sich die Behandlungsaussichten von Magenkrebs dramatisch verbessern. Fortgeschrittener Magenkrebs, der auch mit konventionellen Kontrastmitteln diagnostiziert werden kann, hat bekanntlich eine sehr schlechte Prognose. I. Laufer et al, Diagnostic Radiology, Vol. 115, Juni 1975, Seiten 569-575.

Bei der Doppelkontrastdarstellung des Magens bzw. der Magenschleimhaut wird beispielsweise durch Verabreichung von Carbonaten der Magen mit Kohlensäure, die aus den Carbonaten freigesetzt wurde, prall gefüllt. Nun wird eine relativ geringe Menge einer hochkonzentrierten Bariumsulfat-Suspension möglichst geringer Viskosität eingeführt. Die Bariumsulfat-Suspension, falls sie ausreichend fliessfähig ist, breitet sich auf der gedehnten Magenwand mit all ihren Ausbuchtungen und Feinstrukturen aus, während das Gas selbst als negatives Kontrastmittel wirkt. Auf den Schleimhautfalten soll derart eine feine Schicht von Bariumsulfat entstehen, welche im Röntgenkontrastbild sichtbar wird, falls die Bariumsulfat-Suspension genügend konzentriert ist. Je höher die Konzentration der Bariumsulfat-Suspension ist, umso dünnere Schichten derselben auf der Magenschleimhaut lassen sich im Röntgenbild noch erkennen. Je fliessfähiger die Suspension ist, umso besser dringt sie in feinste Zwischenräume und Falten ein und umso differenziertere Strukturen werden sichtbar. I. Laufer, Diagnostic Radiology, Vol. 117, Dez. 1975, S. 513-518.

Durch die Anwendung der Doppelkontrastdarstellung konnte die Auffindungsrate von Carcinoma um das Vielfache verbessert werden. Unter optimalen Voraussetzungen lassen sich selbst Carcinoma von wenigen mm bis bloss 1 mm Durchmesser erkennen.

Höchste Konzentration und gleichzeitig eine sehr gute Fliessfähigkeit der verwendeten Bariumsulfat-Suspension über einen breiten pH-Bereich sind demnach die unerlässlichen Hauptvoraussetzungen für gute Doppelkontrastdarstellungen. Der Magensaft, dessen pH über einen weiten Bereich schwanken kann, soll keine Flockungen des Kontrastmittels verursachen. R.E. Miller and J. Skucas, loc. cit.

Auf einem ähnlichen Prinzip beruht auch die Doppelkontrastdarstellung der Darmschleimhaut mit deren Hilfe sich ebenfalls verhältnismässig geringfügige typische Veränderungen im Schleimhautrelief und damit Carcinoma bereits im Frühstadium diagnostizieren lassen.

Für die Doppelkontrastdarstellung des Magens bzw. der Magenschleimhaut geeignete Bariumsulfat-Formulierungen sollen mindestens 200 besser 250 g Bariumsulfat pro 100 ml enthalten.

Aus handelsüblichen, gefällten, Pharmakopoe-konformen Bariumsulfat-Pulvern lassen sich gewöhnlich keine genügend fliessfähigen Formulierungen der geforderten hohen Konzentration herstellen, und zwar unabhängig davon ob man dazu grob gemahlene oder sehr fein gemahlene Präparate verwendet. Vergl. dazu beispielsweise W.B. James, British Journal of Radiology, 51 (1978), Seiten 1020-1022.

Gewisse mineralische Bariumsulfate, beispielsweise aus Süd-Australien geben nach Mahlen auf Korngrössen von etwa 1 bis 30 μm hochkonzentrierte Suspensionen mit ausreichender Fliessfähigkeit. Leider genügen solche Baryte den Reinheitsanforderungen der Arzneimittelbücher im allgemeinen nicht. Sie enthalten gewöhnlich zu viel Schwermetalle. R.E. Miller and J. Skucas, loc. cit.

Es besteht daher ein akutes Bedürfnis für Bariumsulfat hoher Schüttdichte und mit guter Fliessfähigkeit von deren hochkonzentrierten wässerigen Suspensionen.

Es wurde nun gefunden, dass sich sowohl Schüttdichte von Bariumsulfaten als auch die Fliessfähigkeit von wässerigen Suspensionen derselben entscheidend verbessern lassen, wenn man durch Fällung erzeugtes oder gemahlenes mineralisches Bariumsulfat einer Hochtemperaturbehandlung unterwirft. Dabei wird das Bariumsulfat gesintert, was sowohl die Fliessfähigkeit erhöht als auch die Abstände zwischen den Partikeln verkleinert und damit Packung und Dichte erhöhen.

Aus dem durch Hochtemperaturbehandlung erhaltenen Bariumsulfat lassen sich nach Beifügung der üblichen Zusätze Präparate mit höchster Schüttdichte und ausreichender Fliessfähigkeit erhalten. Durch die Hochtemperaturbehandlung, insbesondere von feinkörnigen reinen Bariumsulfaten synthetischen Ursprungs, werden Produkte erhalten, welche eine Schüttdichte von 2.5 bis 3 g/ml und dessen rein wässrige Suspensionen bei einer Konzentration von 200 g pro 100 ml eine Viskosität von weniger als 1 000 mPas aufweisen. (1 mPas entspricht 1 Centipoise.)

Die Fliessfähigkeit der Suspensionen bleibt über einen breiten pH-Bereich von etwa 6 bis 1.2 praktisch konstant. Durch den Eintritt entsprechender Suspension in den Magen entstehen daher keine Flockungen.

Das sind sehr entscheidende Vorteile gegenüber den besten bekannten Präparaten, welche gefälltes und damit reines Bariumsulfat enthalten und die für Doppelkontrastdarstellung der Magenschleimhaut im allgemeinen weniger geeignet sind. Obwohl die Verbesserung der Eigenschaften vor allem bei gefälltem, d. h. synthetischem Bariumsulfat dringend erforderlich ist, lassen sich auch mineralische Bariumsulfate (Baryte) durch die Hochtemperaturbehandlung verbessern.

Die Hochtemperaturbehandlung bewirkt ein Sintern, wobei Partikel ineinander-wachsen und wobei Kanten und Ecken der Partikel eingeschmolzen werden und sich die Oberfläche glättet. Damit wird der Reibungswiderstand zwischen den Partikeln vermindert und das Schüttgewicht erhöht.

Zur Erreichung dieses Effektes ist eine Erwärmung auf 700 bis 1 200 °C erforderlich.

Zwecks Zurückdämmung einer partiellen Thermolyse des Bariumsulfates bei der Hochtemperaturbehandlung, besonders im obern Bereich des genannten Temperaturintervalls, kann das Bariumsulfat vor dem Erhitzen mit einem Sulfat, etwa einem bei höheren Temperaturen flüchtigen oder zersetzlichen Sulfat, beispielsweise mit Ammoniumsulfat oder einem Aminsulfat oder mit einem Alkalisulfat (Natrium- oder Kaliumsulfat) oder mit Magnesium- oder Calciumsulfat imprägniert werden.

Bei Verwendung eines bei höheren Temperaturen flüchtigen oder zersetzlichen Sulfates wie Ammoniumsulfat zur Imprägnierung wird direkt nach der Hochtemperaturbehandlung ein Produkt erhalten, das ohne weitere Behandlung den Anforderungen der US Pharmacopeia XX entspricht und direkt zur Herstellung von Röntgenkontrastmitteln verwendet werden kann.

Bei Verwendung von Alkalisulfaten, Magnesium- oder Calciumsulfat muss nach der Hochtemperaturbehandlung das erhaltene gesinterte Bariumsulfat mit Wasser gewaschen werden.

Wird auf die Imprägnierung mit einem Sulfat verzichtet, so ist ausgiebiges Waschen, eventuell mit Salzsäure, zur Entfernung von in Spuren gebildetem Bariumoxid und anschliessendes Waschen mit Wasser aus Sicherheitsgründen angebracht.

Erhaltene Produkte entsprechen den Anforderungen der US Pharmacopeia XX.

Das Verfahren zur Herstellung von als schattengebende Komponente in Röntgenkontrastmitteln geeignetem Bariumsulfat mit erhöhter Fliessfähigkeit und Schüttdichte besteht demnach darin, dass man gefälltes oder mineralisches Bariumsulfat einer Hochtemperaturbehandlung unterwirft.

Diese Hochtemperaturbehandlung wird bei 700 bis 1 200 °C durchgeführt. Der bevorzugte Temperaturbereich liegt bei 800 bis 1 000 °C.

Man kann dabei das Bariumsulfat vor der Hochtemperaturbehandlung mit einem bei höherer Temperatur flüchtigen oder zersetzlichen Sulfat, einem Alkalisulfat oder mit Magnesium- oder Calciumsulfat, imprägnierten.

Man kann aber auch reines Bariumsulfat mit einem Ammoniumsulfat imprägnieren und danach einer Hochtemperaturbehandlung bei 800 bis 1 000 °C unterwerfen.

Gegenstand der Erfindung ist bei 700 bis 1 200 °C, vorzugsweise bei 800 bis 1 000 °C gesintertes Bariumsulfat mit einer Schüttdichte von mindestens 2.5 g/ml, dessen rein wässrige Suspension bei einer Konzentration von 200 g pro 100 ml eine Viskosität von höchstens 1 000 mPas aufweist, zur Verwendung als Röntgenkontrastmittel.

Ein weiterer Gegenstand der vorliegenden Erfindung sind Röntgenkontrastmittel, insbesondere geeignet zur Doppelkontrastdarstellung des Magens bzw. der Magenschleimhaut, welche dadurch gekennzeichnet sind, dass sie pro 100 ml mindestens 200 g durch Hochtemperaturbehandlung gesintertes Bariumsulfat sowie bis zu 3 Gewichtsprozent Hilfsstoffe zur Verbesserung des Geschmacks, des Fliessverhaltens, der Stabilität und zur Herabsetzung der Grenzflächenspannung enthalten.

Als Ausgangsmaterial für das Verfahren wird im Handel erhältliches Bariumsulfat verwendet, das den Vorschriften der US Pharmacopeia XX entspricht. Die Korngrösse des Ausgangsmaterials, die sich bei der Hochtemperaturbehandlung vergrössert, soll möglichst gering sein und etwa 1 bis 30 μm, vorzugsweise 1 bis 5 μm, betragen.

Die durch die Hochtemperaturbehandlung erhaltenen Präparate werden hinsichtlich ihrer Viskosität, Fliessfähigkeit, Schüttdichte, Korngrössenverteilung und ihres Aussehens bei 1 000-, 5 000- und 50 000-facher Vergrösserung verglichen.

Beispiele für Hochtemperaturbehandlung von Bariumsulfat und Vergleich der Eigenschaften der dabei erhaltenen Präparate :

## Beispiel 1

Eine wässerige Bariumsulfat-Suspension enthaltend 214 % $BaSO_4$ (Korngrösse 8-10 μm) g/v wird mit Natriumhydroxid versetzt, bis dessen Konzentration 8.5 % NaOH g/v entspricht.

Die erhaltene Suspension wird 3 Stunden bei 80 °C vorgetrocknet und danach im Muffelofen während 90 Minuten auf 800 °C gehalten.

Nach dem Abkühlen wird das Bariumsulfat zerkleinert, gemahlen und durch ein Sieb mit einer Maschenweite von 396 μm gesiebt. Das gesiebte Produkt wird in 10 %iger Salzsäure zu einer Suspension von 45 % Bariumsulfat g/v angerührt und 90 Minuten bei 80 °C gerührt. Nach dem Abkühlen wird das Bariumsulfat abfiltriert und so oft mit Wasser gewaschen, bis sich im Waschwasser keine Chlor-ionen mehr nachweisen lassen. Das gewaschene Bariumsulfat wird bei 80 °C getrocknet und gesiebt.

Erhaltenes Bariumsulfat entspricht den Anforderungen von USP XX.

## Beispiel 2

Bariumsulfat der Korngrösse 1 bis 3 μm wird mit 0.5 %igem wässerigem Ammoniumsulfat angefeuchtet. Die erhaltene Paste wird während 1 Stunde im Muffelofen auf 900 °C erhitzt.

Nach dem Abkühlen wird das produkt zerkleinert, gemahlen und durch ein Sieb mit der maschenweite 138 μm gesiebt.

Erhaltenes Bariumsulfat entspricht den Vorschriften der USP XX.

## Beispiel 3

Bariumsulfat-Pulver von der Korngrösse 1 bis 3 μm wird im Muffelofen während 90 Minuten auf 900 °C erhitzt.

Nach dem Abkühlen wird das Produkt zerkleinert, gemahlen und gesiebt. Eine wässerige Suspension des so erhaltenen Produktes wird während 90 Minuten bei 80 °C gerührt, anschliessend filtriert und so lange mit Wasser gewaschen, bis sich mit Schwefelsäure im Filtrat keine Bariumionen mehr nachweisen lassen.

Das erhaltene Bariumsulfat wird getrocknet und gesiebt. Es entspricht den Anforderungen der USP XX.

Eigenschaften der erhaltenen Produkte :

Folgende charakteristische Daten wurden ermittelt : Viskosität, Schüttdichte und Aussehen unter dem Mikroskop.

Die Fliessfähigkeit wässriger Suspensionen von Bariumsulfat wurde bestimmt nach der American National Standard Methode DM 1200 der American Society for Testing Materials (ASTM) mittels FORD Viskositäts-Becher Nr. 4. Gemäss dieser Methode wird die Ausflusszeit der Suspension aus dem standardisierten Becher mit bestimmter Oeffnung in Sekunden gemessen.

### Tabelle 1

| $BaSO_4$ Korngrösse Behandlung | Fliessfähigkeit bei 25 °C | | |
|---|---|---|---|
| | Konzentration in % (g/v) | Zeit des Ausfliessens aus dem FORD-Becher Nr. 4 in Sekunden | |
| | | pH ∼ 7 | pH 1.2 |
| 8 - 10 μm unbehandelt | 200[)1] | halbfeste Paste | halbfeste Paste |
| nach Beispiel 1 | 250 | 30" | 30" |
| 1 - 3 μm unbehandelt | 90[)1] | halbfeste Paste | halbfeste Paste |
| nach Beispiel 2 | 250 | 31" | 25" |
| nach Beispiel 3 | 250 | 28" | 20" |

[)1] höchste mögliche Konzentration für eine rührbare Suspension

Viskosität bestimmt mit Rotationsviskosimeter nach Brookfield

Tabelle 2

| Konzentration von BaSO$_4$ in % g/v | Viskosität in Milli-Pascal-Sekunden (mPas) (1 mPas entspricht 1 Centipoise (cP)) | | | |
|---|---|---|---|---|
| | nach unbehandelt | nach Beispiel 1 | nach Beispiel 2 | nach Beispiel 3 |
| **Korngrösse 8 - 10 µm** | | | | |
| 150 | 660 | 100 | | |
| 200 | » 2000 [1] | 450 | | |
| 250 | | 1800 | | |
| **Korngrösse 1 - 3 µm** | | | | |
| 50 | 600 | | | |
| 100 | » 2000 [2] | | < 50 | 50 |
| 150 | | | 140 | 160 |
| 200 | | | 400 | 650 |
| 250 | | | 2200 | > 2000 [3] |

[1] 180 % (g/v) BaSO$_4$ = 1 900 mPas
[2] 80 % (g/v) BaSO$_4$ = 1 500 mPas
[3] 230 % (g/v) BaSO$_4$ = 1 700 mPas

Schüttdichte (Schüttgewicht) von Bariumsulfat-Pulvern

Tabelle 3

| Korngrösse in µm | Schüttdichte in g/ml (nach DIN 53194) unbehandelt | Beispiel 1 | Beispiel 2 | Beispiel 3 |
|---|---|---|---|---|
| 8 - 10 | 2 | 2.71 | | |
| 1 - 3 | 1.05 | | 2.53 | 2.71 |

Korngrössenverteilung

Tabelle 4

| Grösse in µm | Korngrössenverteilung in % g/g | | | | |
|---|---|---|---|---|---|
| | unbeh. 8-10 µm | Beispiel 1 | unbeh. 1-3 µm | Beispiel 2 | Beispiel 3 |
| 20 | 3.7 | 20 | | 3 | 27.61 |
| 20 - 15 | 9.6 | 29.8 | | 1.6 | 15.28 |
| 15 - 10 | 28.1 | 27.4 | | 3.1 | 18.92 |
| 10 - 5 | 34.7 | 15.4 | | 19.1 | 22.19 |
| 5 - 3 | 10.1 | 3.6 | 2.4 | 38 | 8.93 |
| 3 | 13.7 | 3.7 | 97.6 | 35.2 | 7.06 |

5

Aussehen

Das Aussehen der Bariumsulfat-Partikel wurde bei 1 000-, 5 000- und 50 000-facher Vergrösserung beurteilt. Es wird eine Erhöhung der Zahl grösserer Partikel festgestellt, die zweifellos durch Einsintern von kleinen Kristallsplittern entstanden sind.

Ausserdem wird eine Abrundung der Kanten und Ecken sowie eine gewisse Glättung der Oberflächen festgestellt.

Die sehr erhebliche Verbesserung von Bariumsulfaten hinsichtlich ihrer Eignung als schattengebende Komponente in Röntgenkontrastmitteln kommt in den vorstehenden Daten deutlich zum Ausdruck. Das Schüttgewicht wird erhöht und die Viskosität wässriger Suspensionen stark vermindert.

Beispiele 4-7

Bariumsulfat-Pulver der Korngrösse 1 bis 3 μm werden mit 1-%igen wässrigen Lösungen von

4. Bis(dimethylammonium)sulfat,
5. Natriumsulfat,
6. Kaliumsulfat oder
7. Magnesiumsulfat

befeuchtet. Die erhaltenen Pasten werden getrocknet und im Muffelofen 1 Stunde auf 900 °C erhitzt. Nach dem Abkühlen wird das Produkt jeweils zerkleinert, gemahlen, gesiebt und ausgiebig mit Wasser gewaschen. Beim Beispiel 4 kann auf das Waschen verzichtet werden.

Beispiel 8

Bariumsulfat-Pulver der Korngrösse 1 bis 3 μm wird mit einer 1-%igen Suspension von Calciumsulfat zu einer homogenen steifen Paste angerührt und 1 h auf 900 °C erhitzt. Nach dem Abkühlen wird wie nach der im Beispiel 3 beschriebenen Methode aufgearbeitet. Das Produkt enthält Spuren von Calciumsulfat.

Beispiele 9-17

Bariumsulfat-Pulver der Korngrösse 1 bis 3 μm werden im Muffelofen auf 600, 700, 750, 800 und 1 050 °C erhitzt. Nach dem Abkühlen wird das erhaltene Produkt jeweils zerkleinert, gemahlen und gesiebt und wie im Beispiel 3 beschrieben aufgearbeitet.

Die näheren Bedingungen der Hochtemperaturbehandlung, die Schüttdichte der erhaltenen Produkte sowie die Fliessfähigkeit ihrer rein wässrigen Supsensionen, enthaltend 250 g Bariumsulfat/100 ml im direkt anfallenden pH-Bereich (pH 4-7) und nach Ansäuern auf pH 1.2 (pH des Magensaftes) können der Tabelle 5 entnommen werden.

Tabelle 5

| Hochtemperaturbehandlung | | Eigenschaften der erhaltenen Produkte | | |
|---|---|---|---|---|
| Temperatur | Zeit in | Schüttdichte | Fliessfähigkeit mit FORD-Becher Nr. 4 | |
| °C | h | (DIN 53194) | Auslaufzeiten in Sekunden | |
| | | | pH 4 - 7 | pH 1.2 |
| 9. 600 | 4 | 1.15 | Paste | Paste |
| 10. 700 | 2 | 2.30 | 16 | 16 |
| 11. 750 | 0.5 | 2.12 | 16 | 16 |
| 12. 750 | 2 | 2.57 | 16 | 16 |
| 13. 750 | 4 | 2.57 | 16 | 16 |
| 14. 800 | 0.5 | 2.57 | 16 | 16 |
| 15. 800 | 1 | 2.57 | 16 | 16 |
| 16. 800 | 2 | 2.65 | 16 | 16 |
| 17. 1050 | 1 | 2.98 | 16 | 14 |

6

Erhitzen auf 600 °C ist offensichtlich ungenügend. Eine ausreichende Sinterung wird selbst nach 4 Stunden nicht erreicht. Bei 800 °C genügt bereits eine Heizdauer von 0.5 Stunden, um ein dichtes, gut fliessfähiges Produkt zu erhalten. Durch Erhitzen auf höhere Temperaturen werden dichtere Produkte mit der hohen Schüttdichte von ~ 3 erhalten.

### Beispiele für die Formulierung von Röntgenkontrastmittel

In der Praxis werden Trockenpräparate, die sich mit wenig Wasser zu einer stabilen Suspension anrühren lassen oder mit Vorliebe wässrige Bariumsulfat-Suspensionen als Fertigpräparate verwendet.

Dies bedingt den Zusatz von Hilfsstoffen zur Verbesserung des Geschmackes, des Fliessverhaltens, der Stabilität und zur Herabsetzung der Grenzflächenspannung. Die Konzentration der Hilfsstoffe soll insgesamt 3 % (g/g) nicht überschreiten, um das Kontrastmittel nicht zu sehr zu verdünnen.

### 1. Kontrastmittelpulver zum Anrühren mit Wasser

Nachfolgend werden 3 Rezepturen für Trockenpräparate aufgeführt. Diese Präparate werden in Beutel, Flaschen oder Büchsen abgepackt in den Handel gebracht.

Vor der Verabreichung an den Patienten werden diese Trockenpräparate mit 45 ml Wasser angerührt, wobei jeweils 100 ml einer gebrauchsfertigen, gut fliessfähigen Suspension gebildet wird, welche sich für die Doppelkontrastdarstellung des Magens bzw. der Magenschleimhaut eignet.

Rezept 1

| | | |
|---|---:|---|
| Bariumsulfat hergestellt nach Beispiel 3 | 250 | g |
| Sorbitol (D-Sorbit) | 2.94 | g |
| Simethicone $(CH_3)_3Si\{OSi(CH_3)_2\}_nCH_3$[1] | 500 | mg |
| hydrolysiertes Carrageenan | 300 | mg |
| Erdbeeraroma | 180 | mg |
| Vanillearoma | 50 | mg |
| Citronensäure | 20 | mg |
| Natriumsalz von Saccharin | 5.9 | mg |

Rezept 2

| | | |
|---|---:|---|
| Bariumsulfat hergestellt nach Beispiel 2 | 250 | g |
| Sorbitol (D-Sorbit) | 2.94 | g |
| hydrolysiertes Carrageenan | 1.1 | g |
| Simethicone (Polidimethylsiloxan, stabilisiert mit Silicondioxid) | 500 | mg |
| Natriumpoliphosphat | 220 | mg |
| Erdbeeraroma | 180 | mg |
| Natriumsulfat | 147 | mg |
| Vanillearoma | 50 | mg |
| Citronensäure | 20 | mg |
| Na-Salz von Saccharin | 5.9 | mg |

Rezept 3

| | | |
|---|---:|---|
| Bariumsulfat hergestellt nach Beispiel 2 | 250 | g |
| Sorbitol (D-Sorbit) | 2.94 | g |
| hydrolysiertes Carrageenan | 730 | mg |
| Simethicone | 500 | mg |
| Natriumsulfat | 370 | mg |
| Kirschenaroma | 180 | mg |
| Natriumdextransulfat (Mol. gew. ~ 5 000) | 160 | mg |
| Vanillearoma | 50 | mg |
| Citronensäure | 20 | mg |
| Aspartam (α-L-aspartyl-L-phenylalanimethylester) | 18 | mg |

[1] MERCK Index, 9th Ed : No. 8374

Die Fliessfähigkeit der nach den Rezepten 1, 2 und 3 erhaltenen Suspensionen als solche und nach Ansäuern auf pH 1.2 (pH des Magensaftes) wurde nach der Methode von FORD mit Becher Nr. 4 bestimmt.

(Siehe Tabelle Seite 8 f.)

0 133 444

| Rezept | Fliessfähigkeit in Sekunden | |
|---|---|---|
| | pH ∿ 5 | pH 1.2 |
| 1 | 21 (pH 4.8) | 21 |
| 2 | 18 (pH 4.9) | 19 |
| 3 | 18 (pH 5.5) | 19 |

Wenn man das Bariumsulfat in den Rezepten 1, 2 und 3 durch ungesintertes Bariumsulfat derselben Quelle ersetzt, so erhält man in allen drei Fällen beim Anrühren mit 45 ml Wasser nicht fliessfähige Pasten, deren Fliessfähigkeit sich mit dem FORD-Becher nicht bestimmen lässt.

2. Gebrauchsfertige Röntgenkontrastmittelsuspension

| | | |
|---|---|---|
| Bariumsulfat hergestellt nach Beispiel 2 | 250 | g |
| Sorbitol (D-Sorbit) | 2.94 | g |
| hydrolysiertes Carrageenan | 730 | mg |
| Simethicone (Polidimethylsiloxan + wenig Silicondioxid) | 500 | mg |
| Natriumsulfat | 370 | mg |
| Erdbeeraroma | 180 | mg |
| Natriumdextransulfat (Mol. gew. ∿ 5 000) | 160 | mg |
| Natriumbenzoat | 118 | mg |
| Vanillearoma | 50 | mg |
| Citronensäure | 20 | mg |
| Natriumsalz von Saccharin | 5.9 | mg |
| Wasser depurata | 48 | ml |

Das Präparat wird verrührt und in Plastikbeutel, Flaschen oder Dosen abgefüllt.

Das Präparat zeigt eine gute Fliessfähigkeit. Die Fliessfähigkeit bestimmt mit dem FORD-Becher Nr. 4 beträgt :

Präparat vom pH 5.5 16 Sekunden Ausflusszeit
Präparat angesäuert auf pH 1.2 16 Sekunden Ausflusszeit

Vergleich mit ungesintertem Bariumsulfat

Mit demselben Rezept unter Verwendung von nicht-gesintertem Bariumsulfat wurde eine nicht fliessfähige Paste erhalten.

3. Weitere Formulierungsbeispiele von gebrauchsfertigen Bariumsulfat-Präparaten, welche für die Doppelkontrastdarstellung des Magens geeignet sind.

Zusammensetzung der Präparate

250 g Bariumsulfat, hergestellt nach Beispiel X, werden mit 3 g Aroma- und Süssstoffen, einem Polysaccharid oder Salz (Y) und mit (Z g) Wasser versetzt und zu einer homogenen Suspension verarbeitet. Die Fliessfähigkeit der erhaltenen Supsensionen als solche und nach Ansäuern auf pH 1.2 (pH des Magensaftes) wurde nach der Methode FORD mit Becher Nr. 4 bestimmt. Auslaufzeiten in Sekunden (Sek.).

Tabelle 6

| Präparat | X | Polysaccharid Y oder Salz | Z g Wasser | Fliessfähigkeit in Sek. | |
|---|---|---|---|---|---|
| | | | | pH ∿ 5 | pH 1.2 |
| A | 2 | Na-cellulose-sulfat 100 mg | 47.5 | 19 | 17 |
| B | 3 | Na-cellulose-sulfat 100 mg | 45 | 23 (pH 5.7) | 18 |

8

Tabelle 6  (Fortsetzung)

| Präparat | X | Polysaccharid Y oder Salz | Z g Wasser | Fliessfähigkeit in Sek. pH ~ 5 | pH 1.2 |
|---|---|---|---|---|---|
| C | 2 | Na-dextransulfat (mittl. Mol.gew. 5000) 500 mg | 47.5 | 15 | 13 |
| D | 3 | Na-dextran-sulfat 500 mg | 45 | 14 (pH 5.2) | 14 |
| E | 2 | hydrolysiertes Carrageenan 500 mg | 47.5 | 16 (pH 4.5) | 15 |
| F | 3 | hydrolysiertes Carrageenan 500 mg | 45 | 18 (pH 4.7) | 16 |
| G | 2 | Natriumcitrat 370 mg | 47.5 | 15 (pH 6.25) | 20 |
| H | 3 | Natriumcitrat 370 mg | 45 | 16 (pH 6.5) | 19 |
| I | 1 | Natriumcitrat 370 mg | 45.6 | 24 (pH 6.5) | 26 |
| K | 2 | Na-pyrophosphat 370 mg | 47.5 | 16 (pH 7) | 26 |
| L | 3 | Na-pyrophosphat 370 mg | 45 | 19 (pH 8.4) | 27 |
| M | 2 | Na-dioctylsulfo-succinat 370 mg | 47.5 | 17 (pH 4.8) | 14 |
| N | 3 | Na-dioctylsulfo-succinat 370 mg | 45 | 24 (pH 5.8) | 12 |
| O | 1 | Na-dioctylsulfo-succinat 370 mg | 45.6 | 31 (pH 7.0) | 26 |

Auch bei den Formulierungsbeispielen zeigt der Vergleich von Präparaten, die aus durch Hochtemperaturbehandlung gesintertem Bariumsulfat hergestellt wurden, mit Präparaten sonst gleicher Zusammensetzung, welche mit entsprechendem nicht gesintertem Bariumsulfat erhalten wurden, dass allein gesintertes Bariumsulfat die Herstellung hochkonzentrierter und zugleich fliessfähiger Röntgenkontrastmittel erlaubt.

Höchste Konzentration und gute Fliessfähigkeit sind die zwei Hauptvoraussetzungen, die an ein für die Doppelkontrastdarstellung des Magens bzw. der Magenschleimhaut geeignetes Röntgenkontrastmittel gestellt werden. Durch ausreichende Schattendichte selbst dünner Schichten und die Eindringunsfähigkeit in feinste Schleimhautfalten können Reliefdarstellungen der Schleimhaut erzeugt werden, welche die Erkennung von Magenkrebs bereits im Frühstadium mit hoher Sicherheit erlaubt. Durch diese Früherkennung kann die schlechte Prognose von Magenkrebs verbessert werden.

Im europäischen Recherchenbericht wird die deutsche Patentschrift 700 065 zitiert. Das dort beschriebene Verfahren betrifft die Behandlung einer Aufschwemmung von Bariumsulfat in Wasser unter Zusatz von wasserlöslichen Sulfaten im geschlossenen Gefäss (unter Druck) auf etwa 210 °C. Obschon eine obere Temperaturgrenze für dieses Verfahren nicht angeführt wird, ist diese offensichtlich gegeben durch die kritische Temperatur des Wassers von + 374 °C. Diese höchstmögliche Temperatur im zitierten Prozess ist bei weitem viel zu niedrig, um gesintertes Bariumsulfat mit erhöhter Fliessfähigkeit und Dichte zu erzeugen. Ein solcher Effekt ist offensichtlich durch Erhitzen von Bariumsulfat in Wasser bzw. wässrigen Sulfaten nach dem Verfahren der deutschen Patentschrift 700 065 nicht zu erzielen.

Die Hochtemperaturbehandlung gemäss vorliegender Erfindung bedeutet erhitzen auf die weit höhere Temperatur reichend von 700 bis 1 200 °C. Das Imprägnieren des Bariumsulfates mit einem Sulfat beim erfindungsgemässen Verfahren dient zur Verhinderung einer partiellen Thermolyse des Bariumsulfates im oberen Bereich des genannten Temperaturintervalls. Dabei wird Bariumsulfat mit einem Sulfat angefeuchtet und die erhaltene Paste im Muffelofen auf 900 °C zum Sintern erhitzt.

Im europäischen Recherchenbericht wird ausserdem auf Chemical Abstracts 84 : 92337a (SU-A-481 544) verwiesen. Das Referat ist unverständlich, da es offensichtlich nicht möglich ist, eine Bariumhydroxid und Natriumsulfat enthaltende wässrige Lösung herzustellen. Gemäß russischer Patentschrift SU-A-481 544 betrifft diese ein Verfahren zur Herstellung von Bariumsulfat, welches offenbar darin besteht, daß man eine Lösung von Bariumhydroxid mit Sulfanilsäure versetzt und den erhaltenen Niederschlag durch Erhitzen auf 1 000 °C in Bariumsulfat umwandelt.

Die ebenfalls zitierte DE-OS 27 03 600 (Fischer) ist bereits auf Seite 2 dieser Anmeldung besprochen worden. Die von Fischer erreichte Schüttdichte von 1,6 bis 1,7 g/ml wird bei der vorliegenden Erfindung mit 2,5 bis 3 g/ml bei weitem übertroffen.

**Patentansprüche**

1. Bariumsulfat, gesintert bei 700 bis 1 200 °C, mit einem Schüttgewicht von mindestens 2,5 g/ml, dessen rein wässrige Suspension bei einer Konzentration von 200 g/100 ml eine Viskosität von höchstens 1 000 mPas aufweist, zur Verwendung als Röntgenkontrastmittel.

2. Bariumsulfat nach Anspruch 1, dadurch gekennzeichnet, dass es bei 800 bis 1 000 °C gesintert wurde.

3. Röntgenkontrastmittel, enthaltend Bariumsulfat gemäß Anspruch 1, geeignet zur Doppelkontrastdarstellung des Magens bzw. der Magenschleimhaut, dadurch gekennzeichnet, dass es pro 100 ml mindestens 200 g durch Hochtemperatur gesintertes Bariumsulfat sowie bis zu 3 Gewichtsprozent Hilfsstoffe zur Verbesserung des Geschmacks, des Fliessverhaltens, der Stabilität und zur Herabsetzung der Grenzflächenspannung enthält.

**Claims**

1. Barium sulphate, sintered at 700 to 1 200 °C, with a bulk density of at least 2.5 g/ml, the purely aqueous suspension of which has a viscosity no greater than 1 000 mPas at a concentration of 200 g/100 ml, for use as an X-ray contrast medium.

2. Barium sulphate according to Claim 1, characterized in that it has been sintered at 800 to 1 000 °C.

3. X-ray contrast medium, containing barium sulphate according to Claim 1, suitable for the double contrast visualization of the stomach or the gastric mucosa, characterized in that it contains at least 200 g per 100 ml of barium sulphate, sintered by a high-temperature treatment, and up to 3 per cent by weight of additives for improving the taste, the flow characteristics, the stability and for reducing the interfacial tension.

**Revendications**

1. Sulfate de baryum fritté entre 700 et 1 200 °C, avec un poids spécifique apparent d'au moins 2,5 g/ml, dont la suspension dans l'eau pure à une concentration de 200 g/100 ml présente au plus une viscosité de 1 000 mPas, pour l'utilisation comme agent de contraste de rayon X.

2. Sulfate de baryum selon la revendication 1, caractérisé en ce qu'il est fritté entre 800 et 1 000 °C.

3. Agent de contraste contenant du sulfate de baryum selon la revendication 1, approprié à l'imagerie en double contraste de l'estomac ou de la muqueuse de l'estomac, caractérisé en ce qu'il contient pour 100 ml au moins 200 g de sulfate de baryum fritté à haute température ainsi que jusqu'à 3 pour cent en poids d'additifs pour améliorer le goût, la fluidité, la stabilité et pour abaisser la tension superficielle.